# EUROPEAN PATENT SPECIFICATION

(11) **EP 3 318 263 B1**
(45) Date of publication and mention of the grant of the patent: **06.10.2021**
(21) Application number: 17198853.8
(22) Date of filing: 27.10.2017
(51) Int. Cl.: A61K 35/741, A61K 35/744, A61K 35/747, A61K 31/375, A61P 17/18, A61P 17/00, A61P 35/00, A61P 37/08

(54) **ANTIOXIDANT COMPOSITION AND COMPOSITION FOR IMPROVEMENT OF SKIN CONDITIONS**
ANTIOXIDATIONSZUSAMMENSETZUNG UND ZUSAMMENSETZUNG ZUR VERBESSERUNG VON HAUTZUSTÄNDEN
COMPOSITION ANTIOXYDANTE ET COMPOSITION POUR L'AMÉLIORATION D'ÉTATS DE LA PEAU

(30) Priority: 03.11.2016 KR 20160146006
(43) Date of publication of application: 09.05.2018
(73) Proprietor: Cell Biotech Co., Ltd., Gyeonggi-do 10003 (KR)
(72) Inventor: CHUNG, Myung Jun, 06544 Seoul (KR)
(74) Representative: Schnappauf, Georg

(56) References cited:
- EP-A1- 2 149 368
- WO-A1-2016/142767
- DE-U1- 20 202 562
- I GARAIOVA ET AL: "Probiotics and vitamin C for the prevention of respiratory tract infections in children attending preschool: a randomised controlled pilot study", EUROPEAN JOURNAL OF CLINICAL NUTRITION, vol. 69, no. 3, 10 September 2014 (2014-09-10), pages 373-379, XP055455843, GB ISSN: 0954-3007, DOI: 10.1038/ejcn.2014.174
- MONTORSI FRANCESCO ET AL: "Effectiveness of a Combination of Cranberries,Lactobacillus rhamnosus, and Vitamin C for the Management of Recurrent Urinary Tract Infections in Women: Results of a Pilot Study", EUROPEAN UROLOGY, ELSEVIER, AMSTERDAM, NL, vol. 70, no. 6, 7 June 2016 (2016-06-07), pages 912-915, XP029779976, ISSN: 0302-2838, DOI: 10.1016/J.EURURO.2016.05.042
- Anonymous: "Review | Probiotic VitC & Glycolic Pumpkin | Andalou Naturals", TheBunnieHole, 21 June 2014 (2014-06-21), XP055455854, Retrieved from the Internet: URL:http://www.pbunniep.com/2014/06/review -probiotic-vitc-glycolic-pumpkin.html [retrieved on 2018-03-01]
- DAWOOD MAHMOUD A O ET AL: "Immune responses and stress resistance in red sea bream,Pagrus major, after oral administration of heat-killedLactobacillus plantarumand vitamin C", FISH AND SHELLFISH IMMUNOLOGY, ACADEMIC PRESS, LONDON, GB, vol. 54, 16 April 2016 (2016-04-16), pages 266-275, XP029571495, ISSN: 1050-4648, DOI: 10.1016/J.FSI.2016.04.017
- TAGANG ALUWONG ET AL: "Amelioration of Hyperglycaemia, Oxidative Stress and Dyslipidaemia in Alloxan-Induced Diabetic Wistar Rats Treated with Probiotic and Vitamin C", NUTRIENTS, vol. 8, no. 12, 5 May 2016 (2016-05-05), page 151, XP055456082, DOI: 10.3390/nu8050151

## Description

### [Technical Field]

The present teaching relates to an antioxidant composition and/or a composition for improving skin conditions.

### [Background Art]

It is generally known that aging is accelerated by intracellular metabolic imbalance, incomplete oxidation, or deficiency of metabolic/redox intermediates, which also cause various diseases such as cancer, heart diseases, mental illness or skin aging. Particularly, the mechanism of skin aging is as follows. First, reactive oxygen species or free radicals are produced by UV rays or external environmental factors, and the cell membrane is attacked by the produced reactive oxygen species or free radicals, and thus the skin is inflamed. The inflamed tissue destroys proteins or genetic materials by physiological mechanisms to cause skin aging such as wrinkle formation. Thus, prevention of all stages that are involved in the skin aging mechanism is a fundamental solution to skin aging. However, a solution having excellent effects has not yet been reported.

In an attempt to prevent aging, studies on antioxidants have been conducted in earnest since 1969 when McCord and Fridovich discovered the enzyme superoxide dismutase (SOD) that eliminates superoxide radicals. In recent years, it has been found that diseases such as aging or adult diseases are also associated with reactive oxygen species. Thus, studies on antioxidants capable of controlling reactive oxygen species have been actively conducted, and particularly, studies on the antioxidant effect of vitamin C have been actively conducted.

L-ascorbic acid (vitamin C) is a typical antioxidant substance together with flavonoids and Carotenoid glutathione, is essential for the human body, and acts to keep reactive oxygen species in the body always at a constant level. However, if reactive oxygen species in the body are increased by external factors such as UV rays, drugs or environmental pollutants (endocrine disrupters), and other various causes, they can destroy tissues, organs, and cells constituting them. Thus, it is also well known that reactive oxygen species cause cell death, disease development, and cell aging, and the degree of oxidation of molecules constituting cells becomes more severe with age. Thus, if the production of free radicals in cells can be reduced or a system capable of repairing oxidative damage caused by free radicals can be induced, aging and diseases caused by oxidative stress can be prevented or treated.

Meanwhile, in recent years, the commercial manufacture and marketing of functional foods (foods which affect functions of the body in a targeted manner so as to bring about positive effects on physiology and nutrition), particularly probioticcontaining foods, has spread from the well-established Japanese niche marketplace into the global marketplace. While a number of probiotic bacteria of human origin are now being exploited commercially, studies have been actively conducted not only on potential applications of such products, but also on improvement of the efficacy and the application of such products to new fields.

Compositions comprising probiotic bacteria and vitamin C are known (DE 202 02 562 U1; WO 2016/142767 A1; EP 2 149 368 A1; Garaiova et al., 2014, Eur J Clin Nutr 69(3): 373-379; Montorsi et al., 2016, Europ Urol 70(6): 912-915; Dawood et al., 2016, Fish Shellfish Immunol 54: 266-275; Aluwong et al., 2016, Nutrients 8(12): 151).

### [Disclosure]

The present invention is defined by the appended claims.

The invention is based on the teaching which will be explained in the following:

### [Technical Problem]

It is an object of the present teaching to provide an antioxidant composition containing, as active ingredients, probiotics and vitamin C.

Another object of the present teaching is to provide a composition for improving skin conditions, which contains, as active ingredients, probiotics and vitamin C.

However, objects which are to be achieved by the present teaching are not limited to the above-mentioned objects, and other objects of the present teaching will be clearly understood by those skilled in the art from the following description.

### [Technical Solution]

The present inventors have conducted studies, and as a result, have found that a composition containing probiotics in addition to vitamin C induces the activity of antioxidant enzyme to exhibit a synergistic effect on removal of reactive oxygen species, compared to a composition containing vitamin C alone, thereby completing the present teaching.

In one embodiment, the present teaching is directed to an antioxidant composition containing, as active ingredients, probiotics and vitamin C.

As used herein, the term "probiotics" may be defined as live microbial food supplements which beneficially affect the host by improving the intestinal microbial balance, or more broadly, as living microorganisms, which upon ingestion in certain numbers, exert health effects beyond inherent basic nutrition. Cocktails of various microorganisms, particularly species of *Lactobacillus* and *Bifidobacterium,* have traditionally been used in fermented dairy products to promote health. However to be effective, said probiotics must not only survive manufacturing processing, packaging and storage conditions, but also then must survive transit through the gastrointestinal tract so the probiotic material remains viable to have a positive health effect.

In the present teaching, the probiotics lactic acid bacteria strains may be cultured by a general culture method for lactic acid bacteria, and recovered by a separation process such as centrifugation. The recovered lactic acid bacteria may be dried by, but not limited to, freeze-drying, and may be used as probiotics.

The kinds of probiotics that are used in the present teaching are not particularly limited. For example, the probiotics may comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp. Preferably, the probiotics may comprise *Enterococcus faecium, Lactobacillus rhamnosus,* or a mixture thereof.

In the present teaching, the probiotics may be contained in an amount of 3 X 10⁹ to 6 X 10⁹ CFU/g based on the total weight of the composition, but are not limited thereto. If the content of the probiotics is less than 3 X 10⁹ CFU/g, the effect of increasing antioxidant effects cannot be obtained, because the content thereof is low, and if the content of the probiotics is more than 6 X 10⁹ CFU/g, the appearance of the composition can be poor.

In the present teaching, the vitamin C may be contained in an amount of 800 to 1200 mg/g based on the total weight of the composition, but is not limited thereto. If the content of vitamin C is less than 800 mg/g, it cannot exhibit an antioxidant effect, and if the content of vitamin C is more than 1200 mg/g, it can cause side effects due to overdose or pose economic problems.

In addition, the antioxidant composition according to the present teaching may be used for cosmetics, foods, drugs or quasi-drugs, but is not limited thereto.

As used herein, the term "antioxidant" means an effect obtained by inhibiting the production of reactive oxygen species. As used herein, the term "reactive oxygen species" means a state in that oxygen is unstable due to free radicals, and thus has strong activity. Reactive oxygen species may be produced by various physical, chemical and environmental factors, including *in vivo* enzyme systems, reduction metabolisms, chemicals, pollutants and photochemical reactions. Moreover, reactive oxygen species are known to act to non-selectively and irreversibly destroy lipids, proteins, sugars and DNA in cells to cause cell aging or various diseases, including cancer. It was reported that when reactive oxygen species are excessive, they cause toxicity *in vivo,* that is, oxidative stress. In addition, when vitamin C is additionally fed to people with vitamin C deficiency through a process of removing free radicals, it can increase physical ability and exercise capacity, and furthermore, can provide the effect of reducing obesity (Korean Journal of Pediatrics Vol. 48. No. 12, 2005).

The composition containing probiotics and vitamin C as active ingredients according to the present teaching significantly increases antioxidant activity to prevent aging to thereby increase physical ability and exercise capacity, and also activate energy metabolism *in vivo* to thereby exhibit a significant synergistic effect on weight loss, compared to a composition containing vitamin C alone.

In another embodiment, the present teaching provides a composition for improving skin conditions, which contains, as active ingredients, probiotics and vitamin C.

The kinds of probiotics that are used in the present teaching are not particularly limited. For example, the probiotics may comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp. Preferably, the probiotics may comprise *Enterococcus faecium, Lactobacillus rhamnosus,* or a mixture thereof.

In the present teaching, the probiotics may be contained in an amount of 3 X 10⁹ to 6 X 10⁹ CFU/g based on the total weight of the composition, but are not limited thereto. If the content of the probiotics is less than 3 X 10⁹ CFU/g, the effect of increasing antioxidant effects cannot be obtained, because the content thereof is low, and if the content of the probiotics is more than 6 X 10⁹ CFU/g, the appearance of the composition can be poor.

In the present teaching, the vitamin C may be contained in an amount of 800 to 1200 mg/g based on the total weight of the composition, but is not limited thereto. If the content of vitamin C is less than 800 mg/g, it cannot exhibit an antioxidant effect, and if the content of vitamin C is more than 1200 mg/g, it can cause side effects due to overdose or pose economic problems.

In addition, the composition for improving skin conditions according to the present teaching may be used for cosmetics, foods or quasi-drugs, but is not limited thereto.

The skin condition that is suppressed and/or improved by the composition of the present teaching comprises, but not limited thereto, fine wrinkles, a rough skin, a dry skin, a skin cancer, a skin allergy, an inflammation of the skin, and a photosensitive dermatosis.

The antioxidant composition or the composition for improving skin conditions according to the present teaching as described above may be used as a pharmaceutical composition.

For use, the pharmaceutical composition of the present teaching may be formulated into oral preparations such as powders, granules, capsules, tablets, aqueous suspensions and the like, and forms such as external preparations, suppositories and sterile injectable solutions, according to conventional methods, but is not limited thereto. The pharmaceutical composition of the present teaching may contain a pharmaceutically acceptable carrier. For oral administration, the pharmaceutically acceptable carrier may include a binder, a lubricant, a disintegrant, an excipient, a solubilizer, a dispersing agent, a stabilizer, a suspending agent, a coloring agent, a flavoring agent and the like. For injectable preparations, the pharmaceutically acceptable carrier may include a buffering agent, a preservative, an analgesic, a solubilizer, an isotonic agent, a stabilizer and the like. For topical administration, the pharmaceutically acceptable carrier may include a base, an excipient, a lubricant, a preservative and the like. The pharmaceutical composition of the present teaching may be formulated into a variety of dosage forms in combination with the aforementioned pharmaceutically acceptable carriers. For example, for oral administration, the pharmaceutical composition may be formulated into tablets, troches, capsules, elixirs, suspensions, syrups, wafers or the like. For injectable administration, the pharmaceutical composition may be formulated as a unit dosage ampoule or a multiple dosage form. In addition, the pharmaceutical composition may also be formulated into solutions, suspensions, tablets, capsules and sustained-release preparations.

Meanwhile, examples of the carrier, excipient, and diluent suitable for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil or the like. In addition, the pharmaceutical composition of the present teaching may further contain a filler, an anti-agglutinating agent, a lubricating agent, a wetting agent, a flavoring agent, an emulsifying agent, a preservative or the like.

Routes of administration of the pharmaceutical composition according to the present teaching include, but are not limited to, oral, intravenous, intramuscular, intra-arterial, intramarrow, intrathecal, intracardiac, transdermal, subcutaneous, intraperitoneal, intranasal, intraintestinal, topical, sublingual or rectal routes. Oral or parenteral administration is preferred. As used herein, the term "parenteral" includes subcutaneous, intracutaneous, intravenous, intramuscular, intraarticular, intrasynovial, intrasternal, intrathecal, intralesional, and intracranial injection or infusion techniques. The pharmaceutical composition of the present teaching may also be administered in the form of suppositories for rectal administration.

The pharmaceutical composition of the present teaching may vary depending on various factors, including the activity of a particular compound used, the patient's age, weight, general health, sex, diet, administration time, administration mode, excretion rate, drug combination and the severity of a particular disease to be prevented or treated. The dose of the pharmaceutical composition may be suitably selected by a person skilled in the art depending on the patient's condition, weight, the severity of the disease, the type of drug, administration mode and period, and the pharmaceutical composition may be administered at a dose of 0.0001 to 50 mg/kg or 0.001 to 50 mg/kg per day. The pharmaceutical composition may be administered once or several times per day. The dose does not limit the scope of the present teaching in any way. The pharmaceutical composition according to the present teaching may be formulated as pills, coated tablets, capsules, liquids, gels, syrups, slurries or suspensions.

In addition, the antioxidant composition and the composition for improving skin conditions according to the present teaching as described above may also be used as cosmetic compositions.

The cosmetic composition may be prepared in the form of skin lotion, nourishing lotion, nourishing essence, massage cream, cosmetic bathing additives, body lotion, body milk, bath oil, baby oil, baby powder, shower gel, shower cream, sun screen lotion, sun screen cream, suntan cream, skin cream, UV blocking cosmetics, cleansing milk, cosmetic hair removers, face and body lotions, face and body creams, skin lightening cream, hand lotion, hair lotion, cosmetic cream, jasmine oil, bathing soap, liquid soap, cosmetic soap, shampoo, hand cleaners, medicinal soap (non-medical), cream soap, facial wash, whole body cleansers, scalp cleansers, hair rinse, tooth whitening gel, toothpastes, or the like. For this, the composition of the present teaching may further contain a solvent or a suitable carrier, excipient or diluent, which is generally used in the manufacture of cosmetic compositions.

The kind of solvent that may further be contained in the cosmetic composition of the present teaching is not particularly limited, and examples of the solvent include water, saline, DMSO, and combinations thereof. Examples of the carrier, excipient or diluent include, but are not limited to, purified water, oil, wax, fatty acids, fatty acid alcohols, fatty acid esters, surfactants, humectants, thickeners, antioxidants, viscosity stabilizers, chelating agents, buffers, lower alcohols, and the like. In addition, if necessary, the composition of the present teaching may contain a whitening agent, a moisturizing agent, vitamin, a UV blocking agent, fragrance, a coloring agent, an antibiotic, an antibacterial agent, and an antifungal agent.

Examples of the oil that may be used in the composition of the present teaching include hydrogenated vegetable oil, castor oil, cottonseed oil, olive oil, palm-kernel oil, jojoba oil, and avocado oil. Examples of the wax that may be used in the composition of the present teaching include beeswax, spermaceti, carnauba wax, candelilla wax, montan wax, ceresin wax, liquid paraffin, and lanolin.

Examples of the fatty acid that may be used in the composition of the present teaching include stearic acid, linoleic acid, linolenic acid, and oleic acid; examples of the fatty acid alcohol include cetyl alcohol, octyl dodecanol, oleyl alcohol, panthenol, lanolin alcohol, stearyl alcohol, and hexadecanol; and examples of the fatty acid ester include isopropyl myristate, isopropyl palmitate, and butyl stearate. Examples of the surfactant that may be used in the composition of the present teaching include cationic surfactants, anionic surfactants and non-ionic surfactants, which are known in the art. A surfactant of natural origin, if possible, is preferable.

In addition, the composition of the present teaching may contain a humectant, a thickener, an antioxidant and the like, which are widely known in the art, and the kinds and amounts of these components are as known in the art.

In addition, the antioxidant composition and the composition for improving skin conditions according to the present teaching as described above may also be used as food compositions.

The food composition may be prepared as various foods, for example, beverages, gums, teas, vitamin complexes, powders, granules, tablets, capsules, confectionery, cakes, bread and the like. The food composition of the present teaching comprises a plant extract having little or no toxicity and side effects, and thus can be used with confidence even when it is administered for a long period of time for preventive purposes.

When the compound of the present teaching is contained in the food composition, it may be added in an amount of 0.1 to 50 wt% based on the total weight.

When the food composition is prepared as a beverage, there is no particular limitation, except that the beverage contains the food composition at the indicated percentage. The beverage may additionally contain various flavorings or natural carbohydrates, like conventional beverages. Examples of the natural carbohydrates include monosaccharides such as glucose, disaccharides such as fructose, polysaccharides such as sucrose, conventional sugars such as dextrin, cyclodextrin or the like, and sugar alcohols such as xylitol, sorbitol, erythritol or the like. Examples of the flavorings include natural flavorings (thaumatin, stevia extracts, such as rebaudioside A, glycyrrhizin, etc.) and synthetic flavorings (saccharin, aspartame, etc.).

In addition, the food composition of the present teaching may contain various nutrients, vitamins, minerals (electrolytes), flavorings such as synthetic flavorings and natural flavorings, colorants, pectic acid and its salt, alginic acid and its salt, organic acids, protective colloidal thickeners, pH adjusting agents, stabilizers, preservatives, glycerin, alcohol, carbonizing agents that are used in carbonated beverages, etc.

Such components may be used individually or in combination. Although the percentage of such additives is not of great importance, it is generally selected in a range of 0.1 to about 50 parts by weight based on 100 parts by weight of the food composition of the present teaching.

In addition, the food composition of the present teaching can be administered orally as a food or nutritional product, such as milk or whey-based fermented dairy product, or as a food supplement or a health functional food. Specifically, foods such as milk-based products, beverages, juices, soups, or foods for children may be exemplified, but are not limited thereto. The "milk-based product" means any liquid or semi-solid milk or whey-based product having a varying fat content. The milk-based product can be, for example, cow's milk, goat's milk, sheep's milk, cream, fullfat milk, whole milk, low-fat milk or skim milk, ultrafiltered milk, diafiltered milk, microfiltered milk, milk recombined from powdered milk or whey through any processing, or a processed product, such as yoghurt, curdled milk, sour milk, sour whole milk, butter milk, other fermented milk products, such as viili, filling of snack bars, etc. Another important group includes milk beverages, such as whey beverages, fermented milks, condensed milks, infant or baby milks; icecream; milk-containing food such as sweets.

In still another embodiment, the present teaching is directed to a method of improving a skin condition associated with oxidative damage comprising administering to a subject in need thereof, an antioxidant composition containing probiotics and vitamin C.

The skin condition that is suppressed and/or improved by the method of the present teaching comprises, but not limited thereto, fine wrinkles, a rough skin, a dry skin, a skin cancer, a skin allergy, an inflammation of the skin, and a photosensitive dermatosis.

In the present teaching, the use of the probiotics in combination with the vitamin C may have a great synergistic effect on weight loss and the activation of antioxidant enzyme, thereby exhibiting an antioxidant effect and a skin condition improving effect.

In the present teaching, the contents regarding the above-described probiotics and vitamin C are as described above with respect to the antioxidant composition and the composition for improving skin conditions, and thus the specific description thereof will be omitted below.

In the preventing or treating method of the present teaching, routes of administration of each composition include, but are not limited to, oral, intravenous, intramuscular, intra-arterial, intramarrow, intrathecal, intracardiac, transdermal, subcutaneous, intraperitoneal, intranasal, intraintestinal, topical, sublingual or rectal routes. Oral or parenteral administration is preferred.

As used herein, the term "parenteral" includes subcutaneous, intracutaneous, intravenous, intramuscular, intraarticular, intrasynovial, intrasternal, intrathecal, intralesional, and intracranial injection or infusion techniques. The composition of the present teaching may also be administered in the form of suppositories for rectal administration.

The composition of the present teaching may vary depending on various factors, including the activity of a particular compound used, the patient's age, weight, general health, sex, diet, administration time, administration mode, excretion rate, drug combination and the severity of a particular disease to be prevented or treated. The dose of the composition may be suitably selected by a person skilled in the art depending on the patient's condition, weight, the severity of the disease, the type of drug, administration mode and period, and the composition may be administered at a dose of 0.0001 to 50 mg/kg or 0.001 to 50 mg/kg per day. The composition may be administered once or several times per day. The dose does not limit the scope of the present teaching in any way.

In the present teaching, the composition may be in the form of capsules, tablets, granules, injectable solutions, ointments, powders or beverages, and the composition may be for use in humans.

For use, the composition of the present teaching may be formulated into oral preparations such as powders, granules, capsules, tablets, aqueous suspensions and the like, and forms such as external preparations, suppositories and sterile injectable solutions, according to conventional methods, but is not limited thereto. The composition of the present teaching may contain a pharmaceutically acceptable carrier. For oral administration, the pharmaceutically acceptable carrier may include a binder, a lubricant, a disintegrant, an excipient, a solubilizer, a dispersing agent, a stabilizer, a suspending agent, a coloring agent, a flavoring agent and the like. For injectable preparations, the pharmaceutically acceptable carrier may include a buffering agent, a preservative, an analgesic, a solubilizer, an isotonic agent, a stabilizer and the like. For topical administration, the pharmaceutically acceptable carrier may include a base, an excipient, a lubricant, a preservative and the like. The composition of the present teaching may be formulated into a variety of dosage forms in combination with the aforementioned pharmaceutically acceptable carriers. For example, for oral administration, the composition may be formulated into tablets, troches, capsules, elixirs, suspensions, syrups, wafers or the like. For injectable administration, the composition may be formulated as a unit dosage ampoule or a multiple dosage form. In addition, the composition may also be formulated into solutions, suspensions, tablets, capsules and sustained-release preparations.

Meanwhile, examples of the carrier, excipient, and diluent suitable for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil or the like. In addition, the composition of the present teaching may further contain a filler, an anti-agglutinating agent, a lubricating agent, a wetting agent, a flavoring agent, an emulsifying agent, a preservative or the like.

### [Advantageous Effects]

The composition containing probiotics and vitamin C as active ingredients according to the present teaching can more effectively induce the activity of antioxidant enzyme, thereby exhibiting an antioxidant effect and a skin condition-improving effect.

In addition, the composition containing probiotics and vitamin C as active ingredients according to the present teaching can more effectively induce antioxidant effects to activate energy metabolism *in vivo,* thereby exhibiting a significant synergistic effect on weight loss.

### [Description of Drawings]

FIG. 1 shows the superoxide dismutase (SOD) level in each white rat administered with probiotics and vitamin C, measured in Test Example 2.
FIG. 2 shows total antioxidant capacity (TAS) values in each white rat administered with probiotics and vitamin C, measured in Test Example 2.
FIG. 3 graphically shows a change in the serum vitamin C level of each white rat administered with probiotics and vitamin C, measured in Test Example 3.
FIG. 4 graphically shows the distribution *of L. rhamnosus* in the feces of white rats as a result of each treatment of Example 1, measured in Test Example 4.
FIG. 5 graphically shows the distribution of *E. facecium* in the feces of white rats as a result of each treatment of Example 1, measured in Test Example 4.

### [Mode for teaching]

Hereinafter, the present teaching will be described in further detail with reference to examples. It will be obvious to those skilled in the art that these examples are for illustrative purposes only.

### Examples

### Preparation Example 1: Preparation of Test Animals

In order to verify the effect of a combination of probiotics and vitamin C on anti-aging and the like, 4-week-old white rats (male, SD rats, Saeronbio, Korea) were purchased. Two of the animals were housed in each polypropylene cage and acclimated for one week while they were allowed access to feed and water *ad libitum.* In addition, the animals were housed at a temperature of 24±2°C and a relative humidity of 40±20% with a 12-hr light/12-hr dark cycle. During the acclimation period, the health of the rats was checked, and among these rats, animals whose activity was not reduced were selected and used in subsequent tests.

### Example 1: Oral Administration of Probiotics and Vitamin C

In order to verify the effects of probiotics and vitamin C on anti-aging and the like in the white rats selected in Preparation Example 1 above, as shown in Table 1 below, each of PBS (G1) which is a negative control, and 5 X 10⁹ CFU of *L. rhamnosus* and *E. facecium* (G2), L-ascorbic acid (G3), and a mixture of G2 and G3, which are test substances, was administered orally to the white rats for 5 weeks, and then the effects were measured as described below.

**Table 1**

| Group | Substance administered | Dose |
|---|---|---|
| G1 (N=5) | PBS | - |
| G2 | *L. rhamnosus* and *E. facecium* | 5 X 10⁹ CFU/head |
| (N=5) | | |
| G3 | L-ascorbic acid | 1000 mg/head |
| (N=5) | | |
| G4 | G2 + G3 | 5 X 10⁹ CFU/head +1000 mg/head |
| (N=5) | | |

### Test Example 1: Weight Loss by Administration of Probiotics and Vitamin C

In order to examine the stability and phenotype of each white rat when the probiotics and the vitamin C were administered to the test animals for 5 weeks in Example 1, the weight change patterns of the white rats during the period from the day before administration to 5 weeks after the start of administration in Example 1 were measured, and the results of the measurement are shown in Table 2 below.

**Table 2**

| | | Change in weight | | | | | |
|---|---|---|---|---|---|---|---|
| | | 0 week | 1 week | 2 weeks | 3 weeks | 4 weeks | 5 weeks |
| G1 (NC) | Mean (g) | 132.4 | 187.0 | 250.2 | 307.5 | 360.3 | 381.2 |
| | STD (g) | 2.5 | 2.7 | 6.3 | 8.4 | 7.8 | 8.6 |
| G2 (LAB) | Mean (g) | 138.7 | 205.0 | 267.2 | 319.4 | 356.8 | 374.6 |
| | STD (g) | 4.5 | 3.4 | 4.8 | 3.3 | 4.9 | 6.6 |
| G3 (Vit C) | Mean (g) | 137.6 | 178.8 | 237.8 | 293.0 | 338.1 | 354.1 |
| | STD (g) | 1.8 | 4.8 | 3.6 | 2.6 | 3.2 | 4.4 |
| G4 (Mixed) | Mean (g) | 134.6 | 184.6 | 236.3 | 288.6 | 331.0 | 344.2 |
| | STD (g) | 4.4 | 10.9 | 6.3 | 6.8 | 9.6 | 9.7 |

As shown in Table 2 above, the white rats of groups G1 to G4 in Example 1 showed a continuous gain in weight.

In addition, groups G2 and G3 administered with probiotics and vitamin C, respectively, showed weights of 374.6 g and 354.1 g, respectively, after 5 weeks, and group G4 administered with the mixture of probiotics and vitamin C showed a weight of 344.2 g.

The above-described results indicate that administration of the mixture of probiotics and vitamin C significantly increases antioxidant activity, thereby exhibiting a synergistic effect on weight loss.

### Test Example 2: Measurement of Activity of Antioxidant Enzyme in Serum of White Rats

In order to examine the change in antioxidant activity by administration of probiotics and vitamin C (group G4) according to the present teaching, antioxidant enzyme activity and serum vitamin levels were measured in the following manner.

Blood was collected from the orbits of the white rats of Example 1. The collected blood was incubated at room temperature for 2 hours, and then centrifuged at 1500x g at 4°C for 15 minutes to separate sera, and the separated sera were stored at - 80°C until use in experiments.

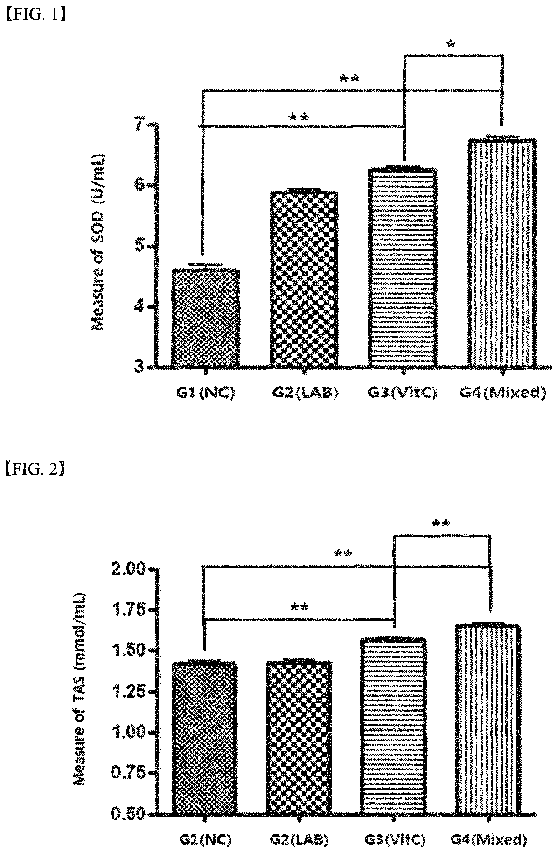

For measurement of the activity of antioxidant enzyme in the separated sera, the activity of superoxide dismutase in the sera was measured using a superoxide dismutase assay kit based on a colorimetry assay, and the results of the measurement are shown in FIG. 1 and Table 3 below.

In order to measure the defense system composed of antioxidant vitamins, nonenzymatic antioxidant substances and antioxidant enzymes, which defend against the toxicity of reactive oxygen species and total antioxidant capacity (TAS) values were measured using a kit of measuring antioxidant activity based on a colorimetry assay. The results of the measurement are shown in FIG. 2 and Table 4 below.

**Table 3**

| | Mean ± SD | | | | *P* Value |
|---|---|---|---|---|---|
| SOD (U/L) | G1 (NC) | G2 (LAB) | G3 (Vit C) | G4 (mixed) | |
| | 4.594±0.1524 | 5.870±0.07317 | 6.248±0.08967 | 6.726±0.1496 | <0.0001 |

**Table 4**

| | Mean ± SD | | | | *P* Value |
|---|---|---|---|---|---|
| TAS (mmol/L) | G1 (NC) | G2 (LAB) | G3 (Vit C) | G4 (mixed) | |
| | 1.418±0.01924 | 1.430±0.02449 | 1.568±0.01708 | 1.655±0.01915 | <0.0001 |

As shown in FIG. 1 and Table 3 above, the activity of antioxidant enzyme (SOD) in group G4 administered with the mixture of probiotics and vitamin C was 6.726 U/L, which was significantly higher than those in groups G2 and G3 administered with probiotics and vitamin C, respectively.

In addition, as shown in FIG. 2 and Table 4 above, group G4 administered with the mixture of probiotics and vitamin C showed the highest total antioxidant capacity (TAS) value (1.655 mmol/L).

The above results suggest that administration of the mixture of probiotics and vitamin C increases the activity of the antioxidant enzyme to thereby exhibit a significant synergistic effect on antioxidant activity, compared to administration of probiotics and vitamin C alone.

### Test Example 3: Measurement of Vitamin C Levels in Sera of White Rats

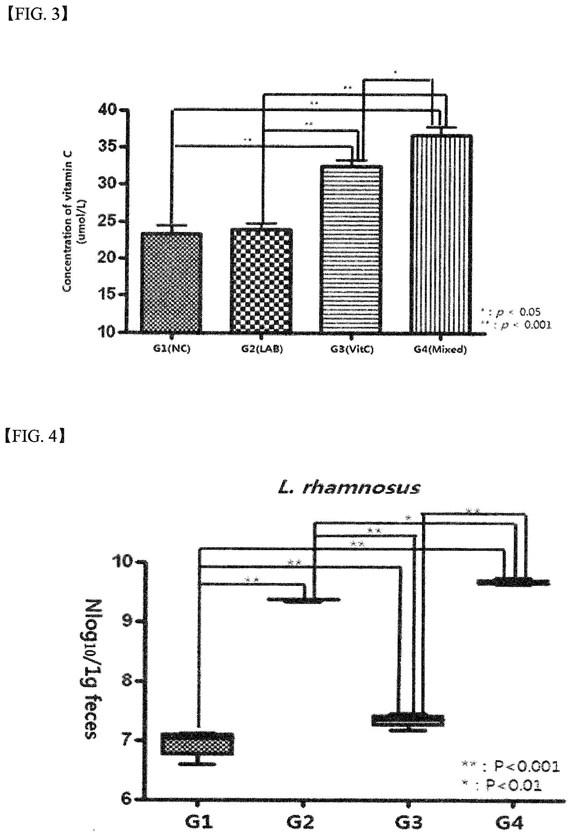

For measurement of vitamin C levels in the sera of the white rats prepared in Example 1, high-performance liquid chromatography (HPLC) analysis was performed using a vitamin C reagent kit, and the results of the analysis are shown in FIG. 3 and Table 5 below.

**Table 5**

| | Mean ± SD | | | | *P* Value |
|---|---|---|---|---|---|
| Vit C (µmol/L) | G1 (NC) | G2 (LAB) | G3 (Vit C) | G4 (mixed) | |
| | 23.35±2.057 | 23.91±1.393 | 32.50±1.289 | 36.60±2.238 | <0.0001 |

As can be seen in FIG. 3 and Table 5 above, co-administration of probiotics and vitamin C according to the present teaching showed the highest serum vitamin C level (36.60 µmol/L) compared to administration of probiotics and vitamin C alone.

These results suggest that the use of probiotics and vitamin C in combination according to the present teaching increases vitamin C absorption in blood by the probiotics to thereby exhibit a synergistic antioxidant effect, compared to administration of probiotics and vitamin C alone.

### Test Example 4: Change in Distribution of Probiotics in Intestines of White Rats

In order to examine the change in intestinal microorganisms by administration of probiotics and vitamin C according to the present teaching, expression of genes specific for *L. rhamnosus* and *E. faecium* in the test groups (G1 to G4) prepared in Example 1 was measured.

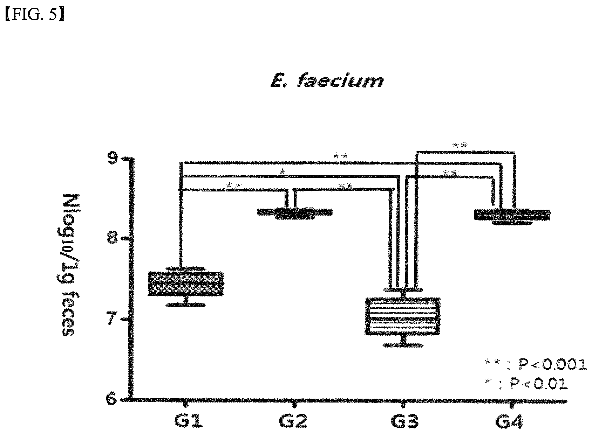

At 5 weeks after oral administration in Example 1, intestinal feces were isolated from the rats of G1 to G4, and DNA was extracted using an MP bio stool kit according to the manufacturer's protocol. Using the extracted DNA, real-time polymerase chain reaction (PCR) was performed using primers specific for *L. rhamnosus* and *E. faecium.* The results are shown in FIGS. 4 and 5 and Tables 6 and 7 below.

**Table 6**

| | Mean ± SD | | | | *P* Value |
|---|---|---|---|---|---|
| *L. rhamnosus* (Nlog₁₀/l g feces) | G1 (NC) | G2 (LAB) | G3 (Vit C) | G4 (mixed) | |
| | 6.950±0.2130 | 9.360±0.01732 | 7.340±0.09975 | 9.664±0.03975 | <0.0001 |

**Table 7**

| | Mean ± SD | | | | *P* Value |
|---|---|---|---|---|---|
| *E. faecium* (Nlog₁₀/l g feces) | G1 (NC) | G2 (LAB) | G3 (Vit C) | G4 (mixed) | |
| | 7.436±0.1641 | 8.334±0.03209 | 7.032±0.1134 | 8.310±0.05612 | <0.0001 |

As can be seen in FIG. 4 and Table 6 above, the distributions *of L. rhamnosus* in group G4 administered with the mixture of probiotics and vitamin C and in group G2 administered with probiotics alone were 9.664 and 9.360 Nlog₁₀/l g feces, respectively. Moreover, as can be seen in FIG. 5 and Table 7 above, the distributions of *E. faecium* in group G4 administered with the mixture of probiotics and vitamin C and in group G2 administered with probiotics alone were 8.310 and 8.334 Nlog₁₀/1 g feces, respectively.

These results suggest that even when the mixture of probiotics and vitamin C according to the present teaching is administered, the antioxidant effect of the vitamin C can be significantly increased while the vitamin C does not adversely affect the distribution of the probiotics.

Although the preferred embodiments of the present teaching have been described in detail above, it will be obvious to those skilled in the art that the scope of the present teaching is not limited to these embodiments and that various modifications and changes are possible without departing from the technical spirit of the present teaching.

## Claims

1. A composition comprising vitamin C and probiotics consisting of *Enterococcus faecium* and *Lactobacillus rhamnosus* as active ingredient.

2. The composition of claim 1, wherein the probiotics are contained in an amount of 3 X 10⁹ to 6 X 10⁹ CFU/g based on the total weight of the composition.

3. The composition of claim 1 for use in treating a skin condition associated with oxidative damage by increasing the vitamin C absorption in the body, wherein the skin condition associated with oxidative damage is selected from the group consisting of a skin cancer, a skin allergy, an inflammation of the skin, and a photosensitive dermatitis.

4. Use of the composition of claim 1 in the treatment of a skin condition associated with oxidative damage, wherein the skin condition comprises any one selected from the group consisting of fine wrinkles, a rough skin, and a dry skin,
with the proviso that methods of treatment of the human or animal body by therapy are not encompassed.

## Patentansprüche

1. Zusammensetzung, die Vitamin C und Probiotika, bestehend aus *Enterococcus faecium* und *Lactobacillus rhamnosus,* als aktiven Inhaltsstoff umfasst.

2. Zusammensetzung nach Anspruch 1, wobei die Probiotika in einer Menge von 3 X 10⁹ bis 6 X 10⁹ CFU/g basierend auf dem Gesamtgewicht der Zusammensetzung enthalten sind.

3. Zusammensetzung nach Anspruch 1 zur Verwendung bei der Behandlung eines Hautzustandes, der mit oxidativem Schaden assoziiert ist, durch Erhöhung der Vitamin-C-Absorption in dem Körper, wobei der Hautzustand, der mit oxidativem Schaden assoziiert ist, ausgewählt ist aus der Gruppe, bestehend aus einem Hautkrebs, einer Hautallergie, einer Entzündung der Haut und einer photosensitiven Dermatitis.

4. Verwendung der Zusammensetzung nach Anspruch 1 bei der Behandlung eines Hautzustandes, der mit oxidativem Schaden assoziiert ist, wobei der Hautzustand ein jegliches umfasst, das ausgewählt ist aus der Gruppe, bestehend aus feinen Falten, einer rauen Haut und einer trockenen Haut,
mit der Maßgabe, dass Verfahren zur therapeutischen Behandlung des menschlichen oder tierischen Körpers nicht umfasst sind.

## Revendications

1. Composition comprenant de la vitamine C et des probiotiques constitués *d'Enterococcus faecium* et de *Lactobacilllus rhamnosus* comme ingrédient actif.

2. Composition selon la revendication 1, dans laquelle les probiotiques sont contenus dans une quantité de 3 x 10⁹ à 6 x 10⁹ CFU/g sur la base du poids total de la composition.

3. Composition selon la revendication 1, pour une utilisation dans le traitement d'une condition de la peau associée à un dommage oxydatif en augmentant l'absorption de vitamine C dans le corps, dans laquelle la condition de la peau associée à un dommage oxydatif est choisie dans le groupe constitué d'un cancer de la peau, une allergie cutanée, une inflammation de la peau, et une dermatite photosensible.

4. Utilisation d'une composition selon la revendication 1, dans le traitement d'une condition de la peau associée à un dommage oxydatif, dans laquelle la condition de la peau comprend n'importe quelle condition sélectionnée dans le groupe constitué de rides fines, d'une peau rêche et d'une peau sèche,
à condition que des procédés de traitement du corps humain ou animal par un traitement ne soient pas inclus.
